# EUROPEAN PATENT APPLICATION

(11) **EP 2 191 776 A1**
(43) Date of publication of application: **02.06.2010**
(21) Application number: 08792780.2
(22) Date of filing: 27.08.2008
(51) Int. Cl.: A61B 8/00

(54) **ULTRASONIC DIAGNOSTIC DEVICE**

(30) Priority: 03.09.2007 JP 2007227575
(71) Applicant: Hitachi Medical Corporation, Chiyoda-ku Tokyo 101-0021 (JP)
(72) Inventor: YAMAMOTO, Masa, Tokyo 101-0021 (JP)
(74) Representative: MERH-IP Matias Erny Reichl Hoffmann
(86) International application number: PCT/JP2008/065270
(87) International publication number: WO 2009/031443

(57) **Abstract**

An ultrasonic diagnostic apparatus (1) includes: an ultrasonic probe (14) which transmits/receives an ultrasonic wave to/from an object (13) to be examined; an image processing unit (16) which configures an ultrasonic image according to the ultrasonic reception signal outputted from the ultrasonic probe (14); an image display unit (2) which displays the configured ultrasonic image; a touch panel (3) arranged on the front face of the image display unit (2); a touch position detection unit (5) which detects a touch position on the touch panel (3); a touch state detection unit (6) which detects a touch state on the touch panel; a processing state detection unit (7) which detects the current processing state; and a process decision unit (9) which decides a process to be executed according to at least one of the detected touch position, touch state, and the processing state and executes the process.

## Description

### Technical Field

The present invention relates to an ultrasonic diagnostic apparatus for imaging and displaying ultrasonic images, particularly the ultrasonic diagnostic apparatus comprising a touch panel for operation.

### Background Art

In conventional ultrasonic diagnostic apparatuses, an operator sets a focus position by operating a focus position switch for exclusive purpose. The operator sets the focus position to be at the nearest position to a diagnostic target portion while referring to an ultrasonic image and a focus mark to be displayed on the side thereof.

Also, the ultrasonic diagnostic apparatus has been proposed that displays an operation button on an image display unit on which a touch panel is placed (For example, refer to [Patent Document 1]).

Patent Document 1: JP-A-2006-26256

### Disclosure of the Invention

### Problems to be Solved

However, in the focus setting in conventional ultrasonic diagnostic apparatuses, selective focus positions are not precise, thus the optimal position needs to be determined by switching the focus position several times in the vicinity of the target region. When the previously selected focus point is away from the target region, the number of times for operating the switch must also be increased. Also, since a dedicated key for moving the focus point is at a separate position from an image display unit, the operator must move his/her line of sight from the ultrasonic image while observing in a dark room and looking at a keyboard to move the focus position. The operator also needs to acquire full knowledge of the switching procedure in advance.

In the technique disclosed in Patent Document 1, since an operating button is displayed on an image display unit comprising a touch panel and the operator has to use this displayed operating button (soft switch) for setting a focus position, it does not save much trouble over operating a dedicated operating button (hard switch) thus saves hardly any labor.

Also, mounting of the touch panel on the image display unit affects the display performance of observation images. Since the touch panel is operated directly by touching with a finger tip, there are cases that observation images are not clearly displayed due to adhesion of oil smudge, etc. If the operator pulls the image display unit on which the touch panel for operation is attached toward his/her hand, the image moves to a position inadequate for observation.

The objective of the present invention is to provide an ultrasonic diagnostic apparatuses with improved operability and diagnosis efficiency.

### Means to solve the problem

In order to achieve the above-described objective, the ultrasonic diagnostic apparatus of the first invention comprises:
an ultrasonic probe configured to transmit/receive an ultrasonic wave to/from an object to be examined;
an image processing unit configured to reconstruct an ultrasonic image based on the ultrasonic reception signal outputted from the ultrasonic probe;
an image display unit configured to display the reconstructed ultrasonic image; and
a touch panel provided on the anterior surface of the image display unit,
and is characterized in further comprising:
   touch-position detection unit configured to detect the touch position on the touch panel;
   touch-state detection unit configured to detect the touch state on the touch panel;
   processing-state detection unit configured to detect the current processing state; and
   processing determination unit configured to determine and execute the process to be performed based on at least one of the detected touch position, the touch state or the processing state.

The ultrasonic diagnostic apparatus of the first invention is provided with a touch panel on the anterior surface of the image display unit. The ultrasonic diagnostic apparatus detects the touch position, touch state and processing state on the touch panel, and determines and executes the processing to be performed on the basis of the detected touch position, touch state and processing state. The processes which can be selected and executed are, for example, setting of focusing position, setting of an ROI, setting of a Doppler sample point, forward and backward inversion of Doppler image display, size setting of an ROI, scaling of an image, setting of attributes associated with an object, or setting of parameters. Also, the ultrasonic diagnostic apparatus creates an operation image for supporting the operation on the touch panel based on the detected touch position, touch state and processing state, and display the created image on the image display unit.

In this manner, the ultrasonic diagnostic apparatus of the first invention switches the processing content and images for supporting the operation, whereby improving operability or diagnosis efficiency.

The ultrasonic diagnostic apparatus of the second invention comprises:
an ultrasonic probe configured to transmit/receive an ultrasonic wave to/from an object to be examined;
an image processing unit configured to reconstruct an ultrasonic image based on the ultrasonic reception signals outputted from the ultrasonic probe; and
a first display unit configured to display the reconstructed ultrasonic image;
   characterized in further comprising:
   a second display unit provided separately from the first display unit, configured to display the ultrasonic image;
   a touch panel provided on the anterior surface of the second image display unit,
   touch-position detection unit configured to detect the touch position on the touch panel;
   touch-state detection unit configured to detect the touch state on the touch panel;
   processing-state detection unit configured to detect the current processing state; and
   processing determination unit configured to determine and execute the process to be performed based on at least one of the detected touch position, the touch state or the processing state.

The ultrasonic diagnostic apparatus of the second invention comprises the first image display unit (observation monitor) configured to display the ultrasonic images for observation, and the second image display unit (operation monitor) to which the touch panel is provided for operation.

The ultrasonic diagnostic apparatus of the second invention creates an operation image for supporting the operation based on the detected touch position, touch state and processing state, and displays the created image on the first image display unit and/or the second image display unit. The operation image to be displayed on the first image display unit and the second display unit may be the same or different.

Also, it is desirable to make the position corresponding to the touch position on the touch panel on the second image display unit identifiable and to be displayed on the first image display unit. Also, it is desirable to dispose the second image display unit comprising the touch panel in the vicinity of the operation unit such as a keyboard of the ultrasonic diagnostic apparatus. Also, it is desirable to make the second image display unit comprising the touch panel to be detachable or connectable by a connection wire to the main body of the ultrasonic diagnostic apparatus. Also, it may be configured so that the guide for indicating the correspondence between the touch state and the process is to be displayed on the image display unit comprising the touch panel.

In this manner, the ultrasonic diagnostic apparatus of the second invention is capable of improving operability and operation efficiency, since touch panel operation can be carried out on the operation monitor without influencing the image observation on the observation monitor by providing the operation monitor by comprising the touch panel separately from the observation monitor. Also, since there is no need to include the touch panel on the observation monitor, image quality of display performance in the observation monitor does not have to be deteriorated.

### Effect of the Invention

In accordance with the present invention, the ultrasonic diagnostic apparatus capable of improving operability and display function can be provided.

### Brief Description of the Drawings

Fig. 1 shows a block diagram of ultrasonic diagnostic apparatus 1 related to a first embodiment.
Fig. 2 is a flowchart showing the operation of ultrasonic diagnostic apparatus 1.
Fig. 3 is a flowchart showing the operation of ultrasonic diagnostic apparatus 1 in setting of a focus position.
Fig. 4 shows image display of image display unit 2 in setting of a focus position.
Fig. 5 is a flowchart showing the operation of ultrasonic diagnostic apparatus 1 in setting of a region of interest.
Fig. 6 shows image display of image display unit 2 in setting a region of interest.
Fig. 7 is a flowchart showing the operation of ultrasonic diagnostic apparatus 1 in setting of a Doppler sample point.
Fig. 8 shows image display of image display unit 2 in setting of a Doppler sample point.
Fig. 9 is a flowchart showing the operation of ultrasonic diagnostic apparatus 1 in forward and backward invention of a Doppler image display.
Fig. 10 shows image display of image display unit 2 in forward and backward invention of Doppler image display.
Fig. 11 is a flowchart showing the operation of ultrasonic diagnostic apparatus 1 in setting of the size of a region of interest.
Fig. 12 shows image display of image display unit 2 in setting of the size of a region of interest.
Fig. 13 is a flowchart showing the operation of ultrasonic diagnostic apparatus 1 in enlargement of an image.
Fig. 14 shows image display of image display unit 2 in enlargement of an image.
Fig. 15 is a flowchart showing the operation of ultrasonic diagnostic apparatus 1 in parameter setting.
Fig. 16 shows image display of image display unit 2 in parameter setting.
Fig 17 shows image display of image display unit 2 in ID setting.
Fig. 18 is a block diagram of ultrasonic diagnostic apparatus 180 related to a second embodiment.
Fig. 19 is a flowchart showing the operation of ultrasonic diagnostic apparatus 180.
Fig. 20 shows image display of operation monitor 181 and observation monitor 182 in setting of a region of interest.
Fig. 21 shows image display of operation monitor 181 and observation monitor 182 in rotation and movement of a probe mark.
Fig. 22 shows guide display of touching operation in operation monitor 181.
Fig. 23 shows display of a touch position.
Fig. 24 is an external view of ultrasonic diagnostic apparatus 240.
Fig. 25 is an external view of ultrasonic diagnostic apparatus 250.

### Description of Reference Numerals

1: ultrasonic diagnostic apparatus (the first embodiment), 2: image display unit, 3: touch panel, 4: controller, 5: touch position detection unit, 6: touch state detection unit, 7: processing state detection unit, 8: operation image creation unit, 9: processing determination unit, 10: processing execution unit, 11: storage unit, 12: setting information, 13: object, 14: ultrasonic probe, 15: ultrasonic transmission/reception unit, 16: image processing unit, 46, 61, 81: ultrasonic image (B-mode image), 42, 45: focus mark, 43, 63, 83: diagnostic target region, 62, 65: region of interest, 82, 85: Doppler sample point, 86, 101, 106: Doppler image, 121, 126, 141: region of interest frame, 123, 125: operation image for changing the size of a region of interest, 161: parameter display region, 171: ID display region, 180: ultrasonic diagnostic apparatus (the second embodiment), 181: operation monitor, 182: observation monitor, 183: touch panel, 203, 206: operation image for changing the size of a region of interest, 212, 215: body mark, 213, 216: probe mark, 221-226: guide, 234: touch position mark, 240, 250: ultrasonic diagnostic apparatus, 242, 252: operation monitor, 243, 253: touch panel, 245, 255: display unit, 246, 256: operation unit, 257: cable

### Best Mode for Carrying Out the Invention

The preferred embodiments of the ultrasonic diagnostic apparatus related to the present invention will be described in detail referring to the attached drawings. In the explanation and the attached drawings below, the components having the same functional configuration will be appended with the same reference numerals and the repeated explanation thereof will be omitted.

### <First Embodiment>

### (1. Configuration of ultrasonic diagnostic apparatus 1)

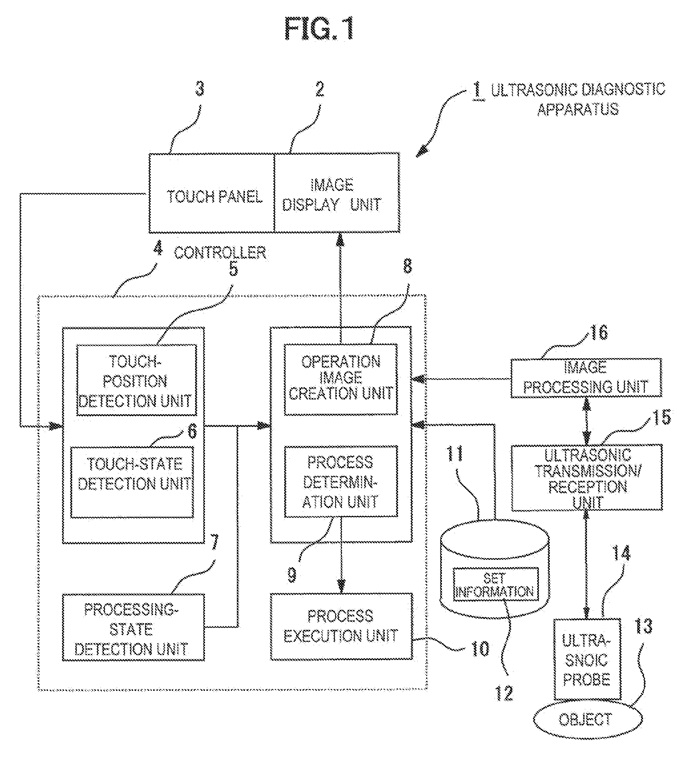

First, configuration of ultrasonic diagnostic apparatus 1 related to the first embodiment will be described referring to Fig. 1.

Fig. 1 is a block diagram of ultrasonic diagnostic apparatus 1 related to the first embodiment.
Ultrasonic diagnostic apparatus 1 comprises ultrasonic probe 14 in which a plurality of transducers for transmitting/ receiving ultrasonic waves to/from object 13 is incorporated, ultrasonic transmission/reception unit 15 for transmitting ultrasonic waves by driving ultrasonic probe 14 and processing the received reflected signals, image processing unit 16 for reconstructing the reflected echo signals into ultrasonic images, image display unit 2 for displaying an operation image for supporting operation such as a focus mark or a region of interest frame along with an ultrasonic image, touch panel 3 provided on the anterior surface of image display unit 2, and controller 4 for executing operation control of the respective components.

Controller 4 comprises touch position detection unit 5, touch state detection unit 6, processing state detection unit 7, operation image creation unit 8, processing determin- ation unit 9 and processing execution unit 10.
Touch position detection unit 5 is for detecting the touch position on touch panel 3. Touch state detection unit 6 is for detecting the touch state on touch panel 3. Touch state is, for example, single touch, double touch, and slide touch. Single touch is the operation of touching on touch panel 3 one time, which is equivalent to the clicking operation by a mouse. Double touch is the operation of touching on touch panel 3 twice in succession, which is equivalent to the double clicking operation by a mouse. Slide touch is the operation to slide while touching on touch panel 3, which is equivalent to the dragging operation by a mouse.

Processing state detection unit 7 is for detecting the processing state of the present ultrasonic diagnostic apparatus 1. Processing state is, for example, "B-mode real time", "CFM mode" and "Doppler mode real time".

Operation image creation unit 8 is for creating an operation image for operation support to be displayed on image display unit 2 along with an ultrasonic image. Processing determination unit 9 is for determining the processing content to be executed by ultrasonic diagnostic apparatus 1 based on the detected touch position, touch state or processing state. Processing execution unit 10 is for executing the determined processing content. The processing to be executed may be, for example, setting of a focus position, setting of a region of interest, setting of a Doppler sample point, forward and backward inversion of Doppler image display, size setting of a region of interest, enlargement and reduction of an image, setting of attribution of an object, and setting of parameters.

Operation image creation unit 8 and processing determination unit 9 are for determining respectively the operation image or the processing to be executed using setting information 12 maintained in storage unit 11.

### (2. Operation of ultrasonic diagnostic apparatus 1)

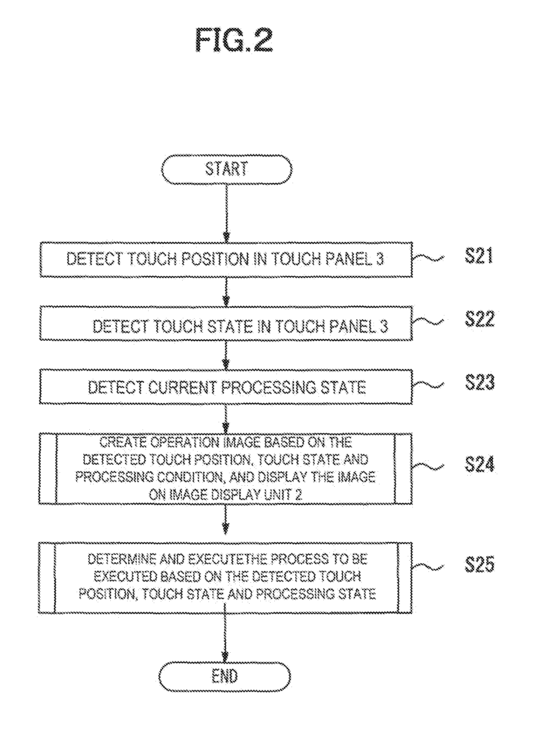

Next, the operation of ultrasonic diagnostic apparatus 1 will be described referring to Fig. 2.
Fig. 2 is a flowchart showing the operation of ultrasonic diagnostic apparatus 1.
Touch position detection unit 5 detects the touch position on touch panel 3 (S21). Touch state detection unit 6 detects the touch state on touch panel 3 (S22). Processing state detection unit 7 detects the current processing state (S23).

Operation image creation unit 8 creates an operation image using the detected touch position, touch state and processing state or setting information 12 maintained in storage unit 11, and displays the created image on image display unit 2 (S24). Processing determination unit 9 determines the processing content to be executed using the detected touch position, touch state and processing state or setting information 12 maintained in storage unit 11, and processing execution unit 10 executes the determined processing (S25).

### (3. Detailed operation and image display of ultrasonic diagnostic apparatus 1)

Next, detailed operation and image display of ultrasonic diagnostic apparatus 1 will be described.

### (3-1. Setting of a focus position)

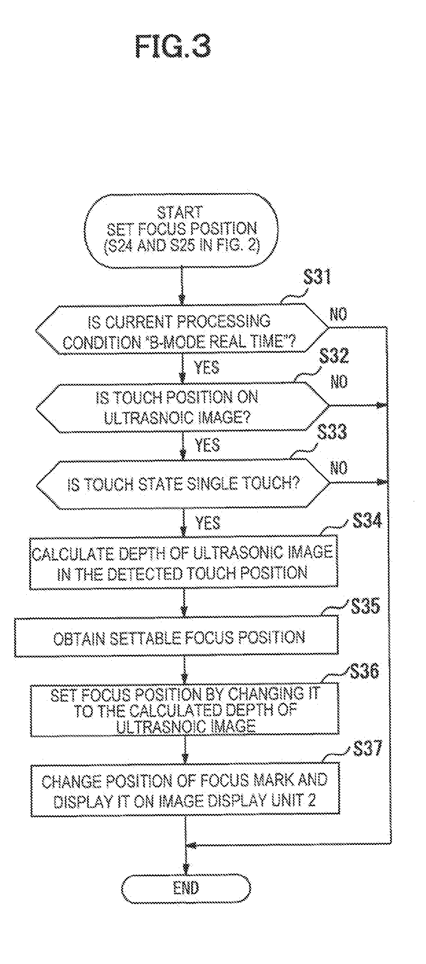

Fig. 3 is a flowchart showing the operation of ultrasonic diagnostic apparatus 1 in setting of a focus position. The flowchart in Fig. 3 is equivalent to the processing of S24 and S26 in Fig. 2.

Controller 4 of ultrasonic diagnostic apparatus 1, in the case that the current processing state is "B-mode real time", touch position is "on an ultrasonic image (B-mode image) and touch state is "single touch" ("YES" in S31, "YES" in S32 and "YES" in S33), calculates the depth of the ultrasonic image at the detected touch position (S34). Controller 4 obtains the settable focus position from setting information 12 maintained in storage unit 11 (S35). Controller 4 changes and sets the focus position to the calculated depth on the ultrasonic image (S36). Controller 4 changes the position of a focus mark, and displays it on image display unit 2 (S37).

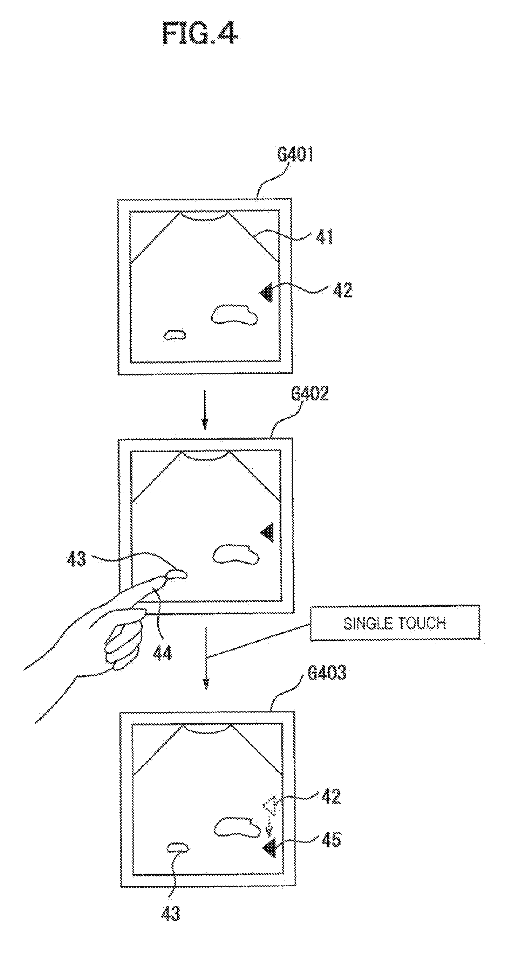

Fig. 4 shows image display of image display unit 2 in setting of a focus position. As shown in screen G401, the focus position is set at the position of focus mark 42 with respect to ultrasonic image 41 (B-mode image). When an operator single-touches the position of diagnostic region 43 via touch panel 3 using fingertip 44 as shown in screen G402, the focus position moves to the position corresponding to the position of diagnostic region 43, and focus mark 45 is displayed as shown in screen G403.

In this manner, in the case that the processing state is "B-mode real time", by directly touching the diagnostic region on the ultrasonic image via the touch panel, the focus position can be changed to the touch position. Also, since the focus position is set at the most suited position for a touch position, there is no need to determine the most suited position by executing the focus position setting operation for a plurality of times.

### (3-2. Setting of a region of interest)

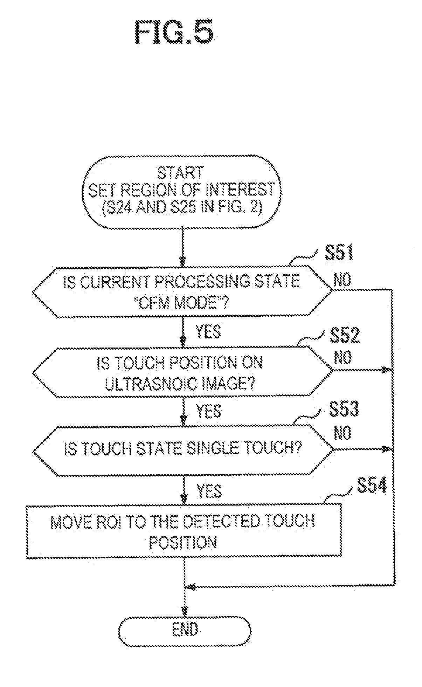

Fig. 5 is a flowchart showing the operation of ultrasonic diagnostic apparatus 1 in setting of a region of interest. The flowchart in Fig. 5 is equivalent to the processing of S24 and S25 in Fig. 2.

Controller 4 of ultrasonic diagnostic apparatus 1, in the case that the current processing state is "CFM (color flow mapping) mode", touch position is "on an ultrasonic image" and the touch state is "single touch" ("YES" in S51, "YES" in S52 and "YES" in S53), the region of interest is moved to the detected touch position (S54).

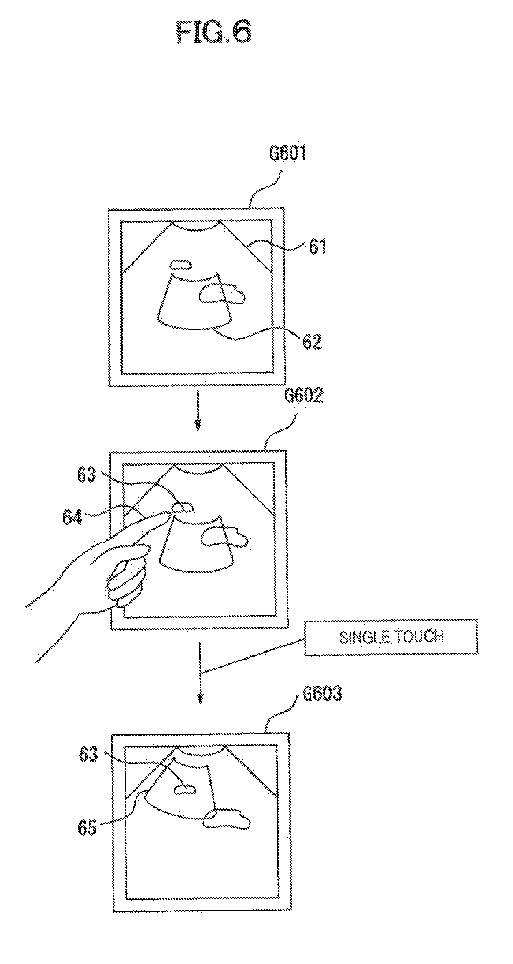

Fig. 6 shows image display of image display unit 2 in setting of a region of interest.
As shown in screen G601, region of interest frame 62 is displayed with respect to ultrasonic image 61. When the operator single-touches the position of diagnostic region 63 via touch panel 3 using fingertip 64 as shown in screen G602, region of interest frame 65 moves to the position corresponding to the position of diagnostic region 63 as shown in screen G603.

In this manner, in the case that the processing state is "CFM mode", by directly touching the diagnostic region on an ultrasonic image via the touch panel, the region of interest can be moved to the touch position.

### (3-3. Setting of a Doppler sample point)

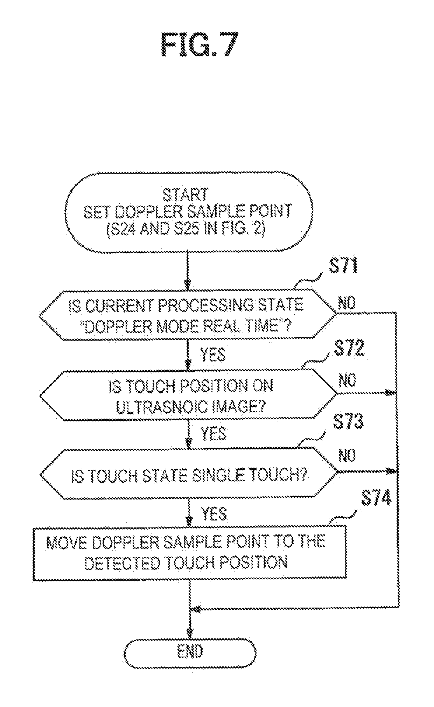

Fig. 7 is a flowchart showing the operation of ultrasonic diagnostic apparatus 1 in setting of a Doppler sample point. The flowchart in Fig. 7 is equivalent to the processing of S24 and S25 in Fig. 2.

Controller 4 of ultrasonic diagnostic apparatus 1, in the case that the current processing state is "Doppler mode real time", the touch position is "on an ultrasonic image (B-mode image)" and the touch state is "single touch" ("YES" in S71, "YES" in S72 and "YES" in S73), the Doppler sample point is moved to the detected touch position (S74).

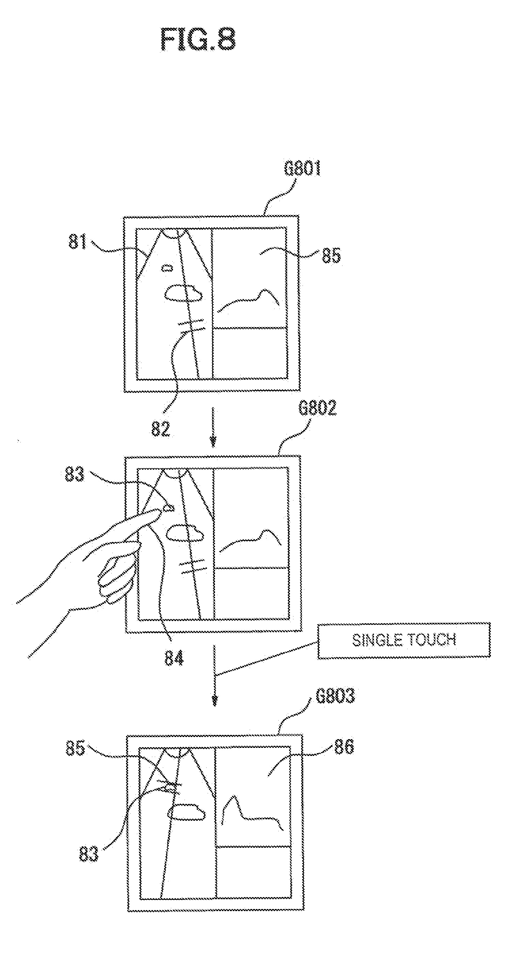

Fig. 8 shows image display of image display unit 2 in setting of a Doppler sample point.

As shown in screen G801, Doppler sample point 82 is displayed with respect to ultrasonic image 81. Further, Doppler image 85 in Doppler sample point 82 is displayed. When the operator single-touches the position of diagnostic region 83 via touch panel 3 using fingertip 84 as shown in screen G803, Doppler sample point 85 is moved to the position corresponding to the position of diagnostic region 83 as shown in screen G803. Further, Doppler image 86 in Doppler sample point 85 is displayed.

In this manner, in the case that the processing state is "Doppler mode real time", the Doppler sample point can be set to the touch position by directly touching the diagnostic region on an ultrasonic image (B-mode image) via the touch panel, the Doppler sample point can be set at the touch position.

### (3-4. Forward and backward inversion of Doppler image display)

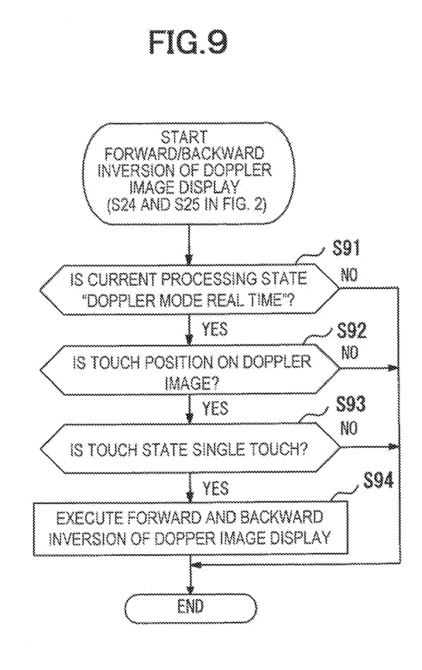

Fig. 9 is a flowchart showing the operation of ultrasonic diagnostic apparatus 1 in forward and backward invention of Doppler image display. The flowchart in Fig. 9 is equivalent to the processing of S24 and S25 in Fig. 2.

Controller 4 of ultrasonic diagnostic apparatus 1, in the case that the current processing state is "Doppler mode real time", the touch position is "on a Doppler image" and the touch state is "single touch" ("YES" in S91, "YES" in S92 and "YES" in S93), executes forward and backward invertion of Doppler image display (S94).

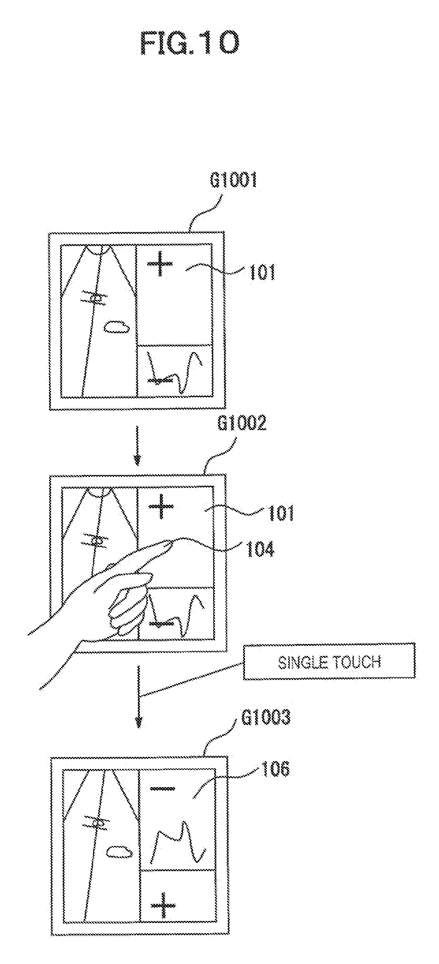

Fig. 10 shows image display of image display unit 2 in forward and backward inversion of Doppler image display.

As shown in screen G1001, Doppler image 101 is displayed. As shown in screen G1002, when the operator single-touches on Doppler image 101 via touch panel 3 using fingertip 104, forward and backward inversion of Doppler image display is executed and Doppler image 106 is displayed.

In this manner, in the case that the processing state is "Doppler mode real time", by touching on the Doppler image via the touch panel, forward and backward inversion of Doppler image display can be executed.

### (3-5. Setting of the size of a region of interest)

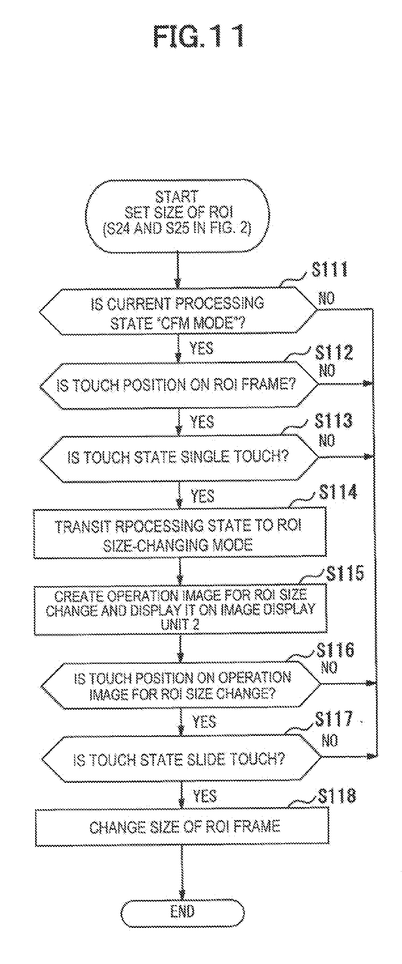

Fig. 11 is a flowchart showing the operation of ultrasonic diagnostic apparatus 1 in setting of a region of interest. The flowchart in Fig. 11 is equivalent to the processing of S24 and S25 in Fig. 2.

Controller 4 of ultrasonic diagnostic apparatus 1, in the case that the current processing state is "CFM (color flow mapping) mode", the touch position is "on a region of interest frame" and the touch state is "single touch" ("YES" in S111, "YES" in S112 and "YES" in S113), the processing state is transit to "region of interest size changing mode" (S114). Controller 4 creates an operation image for changing the region of interest size, and displays the created image on image display unit 2 (S115).

Controller 4 of ultrasonic diagnostic apparatus 1, in the case that the touch position is on an operation image for changing the region of interest size and the touch state is "slide touch" ("YES" in S116 and "YES" in S117), changes the size of the region of interest frame (S118).

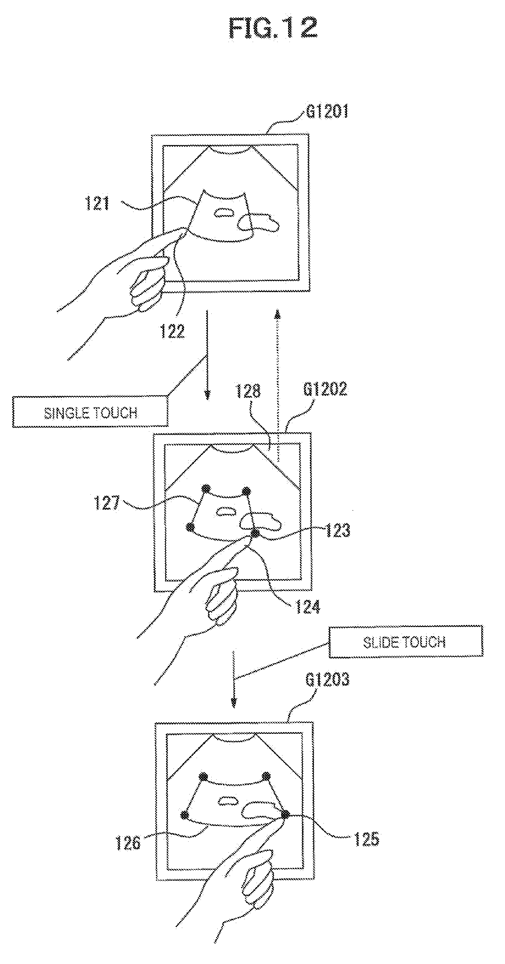

Fig. 12 shows image display of image display unit 2 in setting the size of a region of interest.

As shown in screen G1201, region of interest frame 121 is displayed. As shown in screen G1202, when the operator single-touches on region of interest frame 121 via touch panel 3 using fingertip 122, operation image 123 for changing the size of a region of interest is displayed as shown in screen G1202. Operation image 123 for changing the size of a region of interest is, for example, a slide-touch point (equivalent to the drag point by a mouse). When the operator slide-touches operation image 123 for changing the size of a region of interest via touch panel 3 using fingertip 124, the size of region of interest frame 126 is changed and set to the position of operation image 125 for changing the size of the region of interest, as shown in screen G1203.

In the case that display region 128 other than ultrasonic image 127 is touched in screen G1202, the screen returns to screen G1201.

In this manner, in the case that the processing state is "CFM mode", by touching a region of interest frame via the touch panel, setting of a region of interest size can be executed when "region of interest size changing mode" is set as the processing condition.

### (3-6. Enlargement of an image)

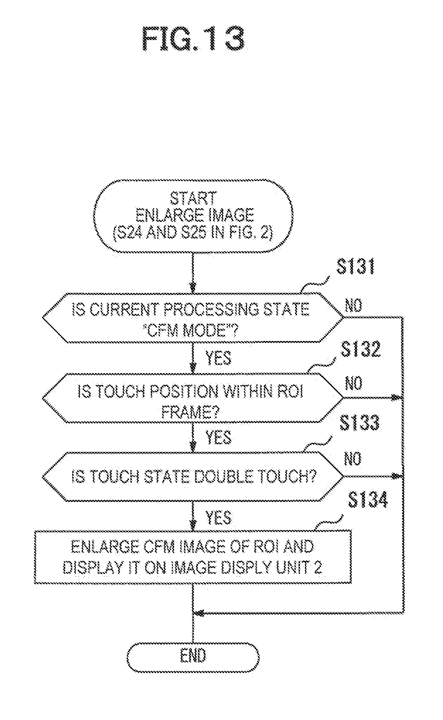

Fig. 13 is a flowchart showing the operation of ultrasonic diagnostic apparatus 1 in enlargement of an image. The flowchart in Fig. 13 is equivalent to the processing of S24 and S25 in Fig. 2.

Controller 4 of ultrasonic diagnostic apparatus 1, in the case that the current processing state is "CFM (color flow mapping) mode", the touch position is within the region of interest frame and the touch state is "double touch" ("YES" in S131, "YES" in S132 and "YES" in S133), enlarges the CFM image of the region of interest and displays the enlarged image on image display unit 2 (S134).

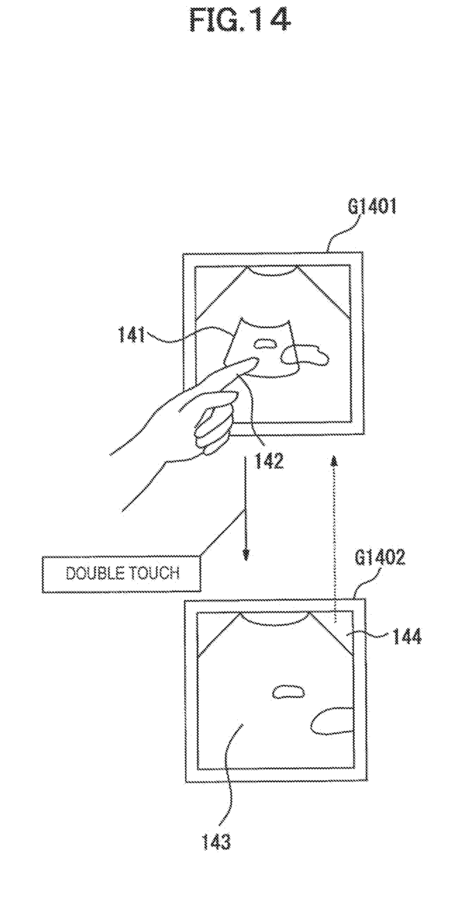

Fig. 14 shows image display of image display unit 2 in enlargement of an image.

Region of interest frame 141 is displayed as shown in screen G1401. As shown in screen G1402, when the operator single-touches within region of interest region frame 141 via touch panel 3 using fingertip 142, CFM image 143 in region of interest frame 141 is enlarged and displayed as shown in screen G1402.

When display region 144 other than CFM image 143 is touched in screen G1402, enlargement display is cancelled and returns to screen G1401.

In this manner, in the case that the processing state is "CFM mode", by touching within the region of interest frame via the touch panel, a CFM image can be enlarged and displayed.

### (3-7. Setting of parameter and ID)

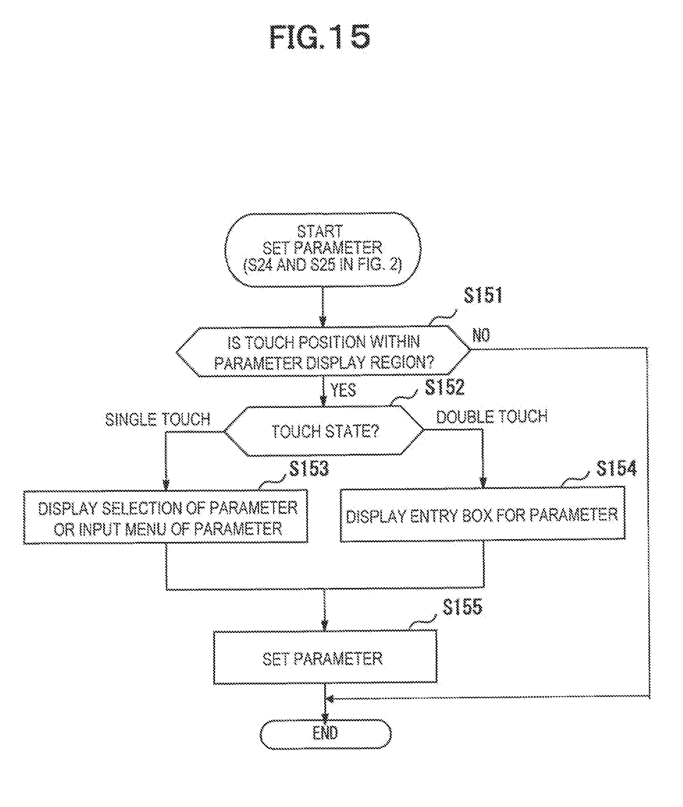

Fig. 15 is a flowchart showing the operation of ultrasonic diagnostic apparatus 1 in setting parameter. The flowchart in Fig. 15 is equivalent to the processing of S24 and S25 in Fig. 2.

Controller 4 of ultrasonic diagnostic apparatus 1, in the case that the touch position is within the parameter display region and the touch state is "single touch" ("YES" in S151 and "single touch" in S152), displays the selection items or input menu of the parameter (S153).

Controller 4 of ultrasonic diagnostic apparatus 1, in the case that the touch position is within the parameter display region and the touch state is "double touch" ("YES in S151 and "double touch" in S152), displays the input column of the parameter (S154).

Controller 4 of ultrasonic diagnostic apparatus 1 sets the parameter selected or inputted in the process of S153 or S154 (S155).

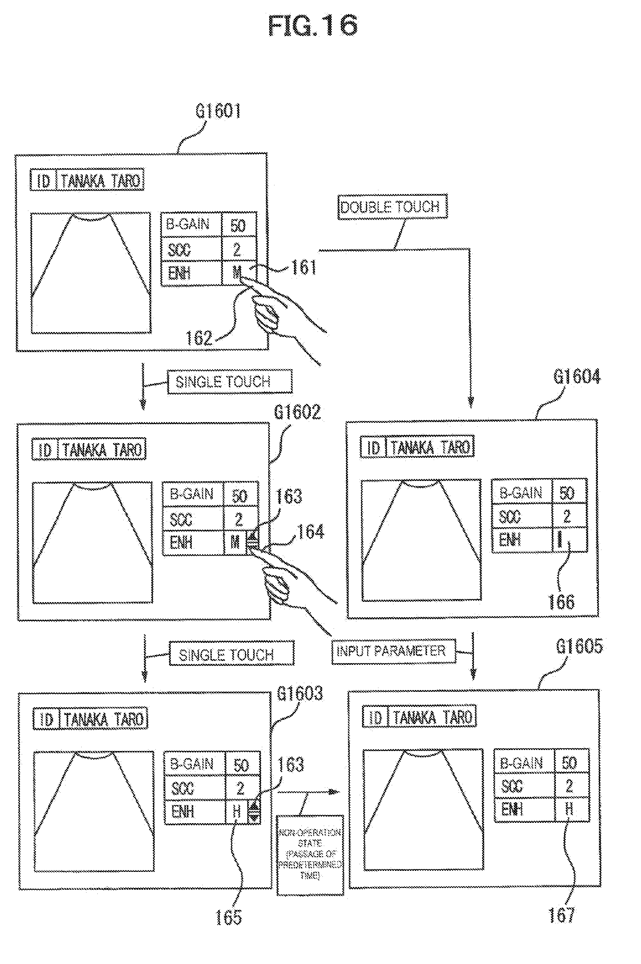

Fig. 16 shows image display of image display unit 2 in setting of parameter.

When the operator single-touches parameter display region 161 using fingertip 162 as shown in screen 1601, buttons 163 ("□""□") for changing parameter values are displayed as shown in screen G1602.

As shown in screen G1603, when the operator single-touches buttons 163 for changing parameter values using fingertip 164, parameter value 165 is changed each time. In screen G1603, when a predetermined time (for example, a several seconds) passes without operation, buttons 163 for changing parameter values disappear and parameter value 167 is set as shown in screen 1605.

In screen 1601, when the operator double-touches parameter display region 161 using fingertip 162, field 166 for inputting parameter values is displayed as shown in screen G1604. When a parameter value is inputted in field 166 for inputting parameter values, parameter value 167 is set as shown in screen 1605.

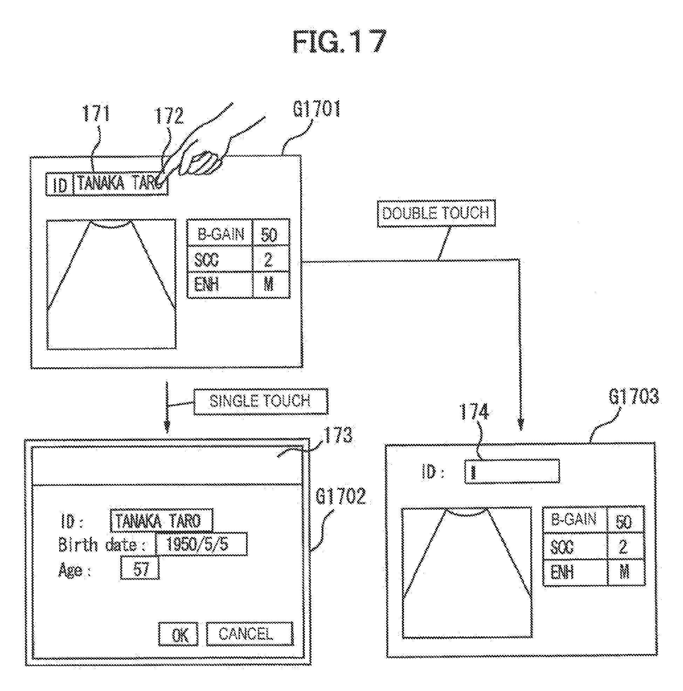

Fig. 17 shows image display of image display unit 2 in setting ID.

In screen G1701, when the operator single-touches ID display region 171 using fingertip 172, ID menu 173 is displayed as shown in screen G1702. By operating ID menu 173, ID or attribute of object 13 are set.

In screen G1701, when the operator double-touches ID display region 171 using fingertip 172, ID input field 174 is displayed as shown in screen G1703. The ID is set based on the content inputted to ID input field 174.

In this manner, setting of parameter or ID can be executed by touching the display region of parameter or ID via the touch panel.

### (4. Effect of the first embodiment)

As described above in detail, the ultrasonic diagnostic apparatus of the first embodiment switches the processing content or images for supporting operation in accordance with the current processing state, touch position or touch state which saves the operation of the key or button for exclusive use, thus is capable of improving operability or diagnosis efficiency.

### <Second Embodiment>

### (Configuration of ultrasonic diagnostic apparatus 180)

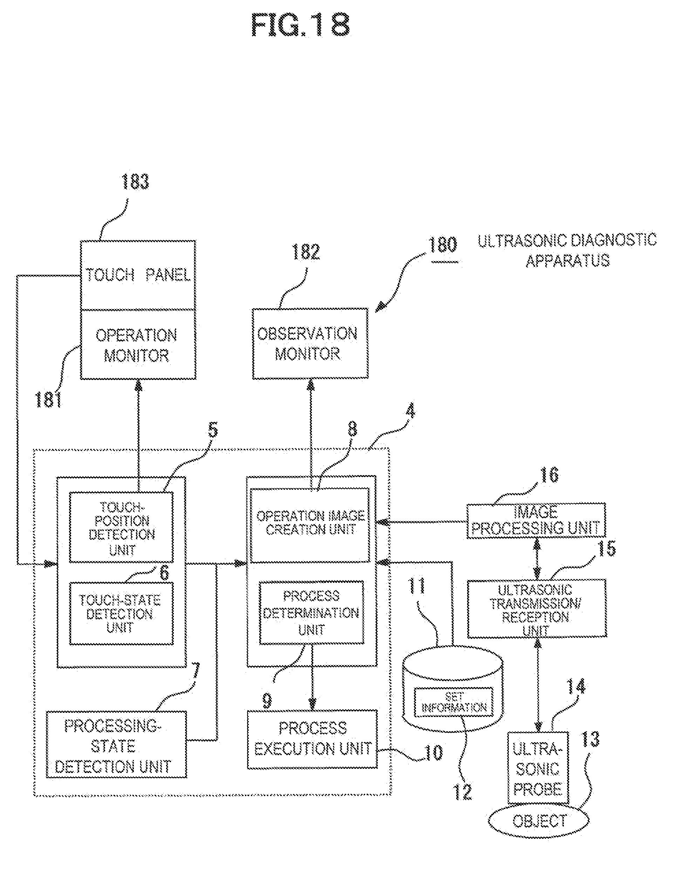

First, the configuration of ultrasonic diagnostic apparatus 180 related to the second embodiment will be described referring to Fig. 18.

Fig. 18 is a block diagram of ultrasonic diagnostic apparatus 180 elated to the second embodiment.

Ultrasonic diagnostic apparatus 180 comprises operation monitor 181 and observation monitor 182 for displaying an operation image for supporting operation such as a focus mark or a region of interest frame along with an ultrasonic image.

While touch panel 3 of ultrasonic diagnostic apparatus 1 in Fig. 1 is provided on image display unit 2, ultrasonic diagnostic apparatus 180 in Fig. 18 comprises operation monitor 181 separate from observation monitor 182, and touch panel 183 is provided on this operation monitor 181. Controller 4, storage unit 11, setting information 12, image processing unit 16, ultrasonic transmission/reception unit 15, and ultrasonic probe 14 in Fig. 18 are the same as in Fig. 1.

The touch panel may be provided not only on operation monitor 181 but also on observation monitor 182. Also, use/disuse of the touch panel may be set as switchable. Also, controller 4, when executing the same display on operation monitor 181 and observation monitor 182, creates one set of image information and outputs it to operation monitor 181 and observation monitor 182. Also, controller 4, when executing different displays on operation monitor 181 and observation monitor 182, creates two sets of image information and outputs them to operation monitor 181 and observation monitor 182 respectively.

### (6. Operation of ultrasonic diagnostic apparatus 180)

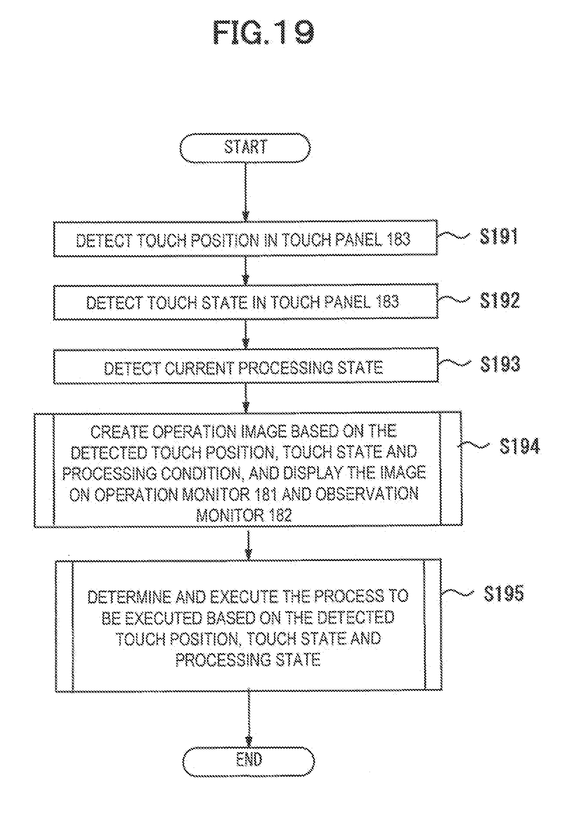

Next, operation of ultrasonic diagnostic apparatus 180 will be described referring to Fig. 19.

Fig. 19 is a flowchart showing the operation of ultrasonic diagnostic apparatus 180.

Touch position detection unit 5 detects the touch position on touch panel 183 (S191). Touch state detection unit 6 detects the touch state on touch panel 3 (S192). Processing detection unit 7 detects the current processing state (S193).

Operation image creation unit 8 creates an operation image using the detected touch position, touch state and processing state or setting information 12 maintained in storage unit 11 and displays the created image on operation monitor 181 and observation monitor 182 (S194). Processing determination unit 9 determines the processing to be executed using the detected touch position, touch state and processing state or setting information 12 maintained in storage unit 11, and processing execution unit 10 executes the determined processing (S195).

### (7. Image display of ultrasonic diagnostic apparatus 180)

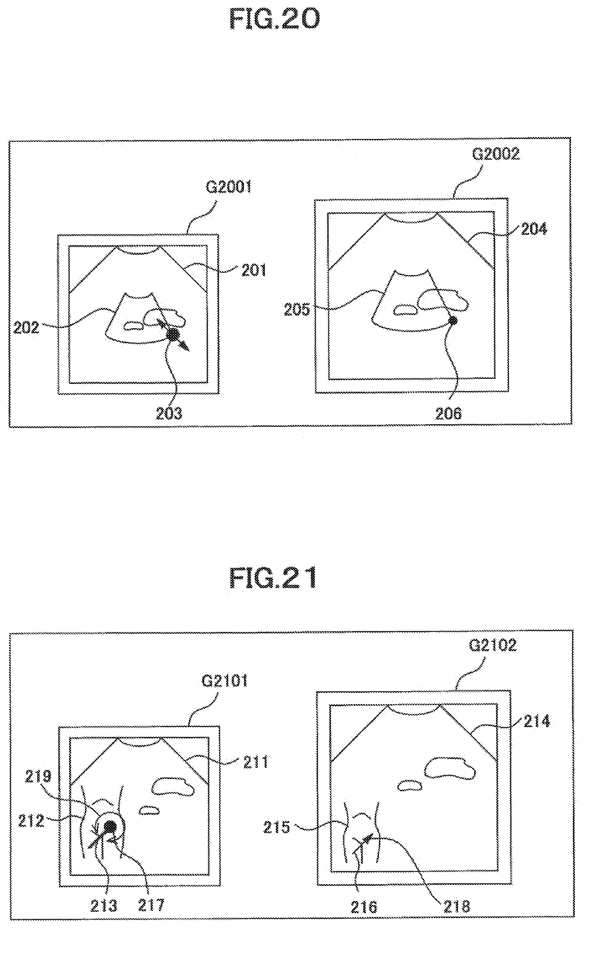

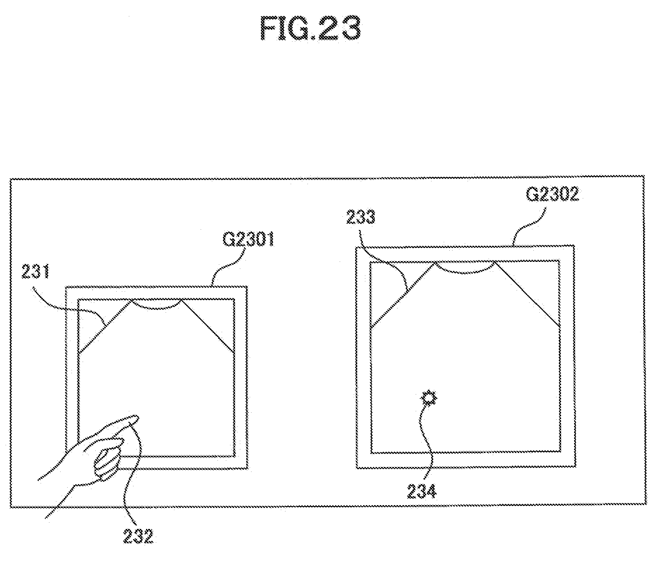

Next, image display in ultrasonic diagnostic apparatus 180 will be described referring to Fig. 20 ∼Fig. 23.

### (7-1. Setting of a region of interest)

Fig. 20 shows image display of operation monitor 181 and observation monitor 182 in setting of a region of interest.

On screen G2001 of operation monitor 181, ultrasonic image 201, region of interest frame 202 and operation image 203 for changing the size of the region of interest are displayed.

On screen G2002 of observation monitor 182, ultrasonic image 204, region of interest frame 205 and operation image 206 for changing the size of a region of interest are displayed.

On operation image 203 of operation monitor 181 and operation image 206 of observation monitor 182 have different formats. Also, the image to be used for the actual touching operation related to changing size of a region of interest is operation image 203 which is provided on operation monitor 181. Operation image 206 of observation monitor 182 is confined to display the screen indicating that the ongoing mode is for changing the size of a region of interest.

As for the format of operation image 203 of operation monitor 181, it is desirable to place importance on operability. For example, it is desirable to make the size of operation image 203 of operation monitor 181 larger than operation image 206 of observation monitor 182, or to configure operation image 203 easy to operate by fingertip-touching. On the other hand, it is desirable to place importance on visibility of ultrasonic image 204 regarding the format of operation image 206 of observation monitor 182. For example, it is desirable to make the size of operation image 206 on observation monitor 182 smaller than operation image 203 on operation monitor 181 to secure the space for display region for ultrasonic image 206.

In this manner, by confining the display on the observation monitor to indicating that the mode is for changing size of the region of interest and by displaying an operation image for changing the size of the region of interest on the operation monitor, it is possible to improve operability related to changing the size of a region of interest without influencing the image observation on the observation monitor.

### (7-2. Rotation and movement of a probe mark)

Fig. 21 shows image display of operation monitor 181 and observation monitor 182 in rotation and movement of a probe mark.

On screen of G2101 of operation monitor 181, ultrasonic image 211, body mark 212 and probe mark 213 are displayed. On screen G2102 of observation monitor 182, ultrasonic image 214, body mark 215 and probe mark 216 are displayed.

When the operator touches body mark 212 via touch panel 183, body mark 212 and probe mark 213 on operation monitor 181 are enlarged and displayed. On the other hand, the display of body mark 215 and probe mark 216 of observation monitor 182 remains the same.

Also, the moving operation by slice touching can be made easier by displaying probe mark 213 of operation monitor 181 with a thicker mark than probe mark 216 of observation monitor 182. Also, rotation operation by sliding touch can be made easier by making apical end 217 of probe mark 213 on operation monitor 181 larger line map (for example, a circle) compared to apical end 218 of probe mark 216 in observation monitor 182. Further, the message can be clearly demonstrated that the rotation operation by slide-touch can be executed, by adding line map 219.

In operation monitor 181, body mark 212 and probe mark 213 return back to the normal display by touching the region other than body mark 212.

In this manner, by making display of the body mark and the probe mark simple on the observation monitor and displaying the body mark and the probe mark in detail on the operation monitor, operability in moving or rotating the probe mark can be improved without influencing the image observation of the observation monitor.

### (7-3. Guide display of touching operation)

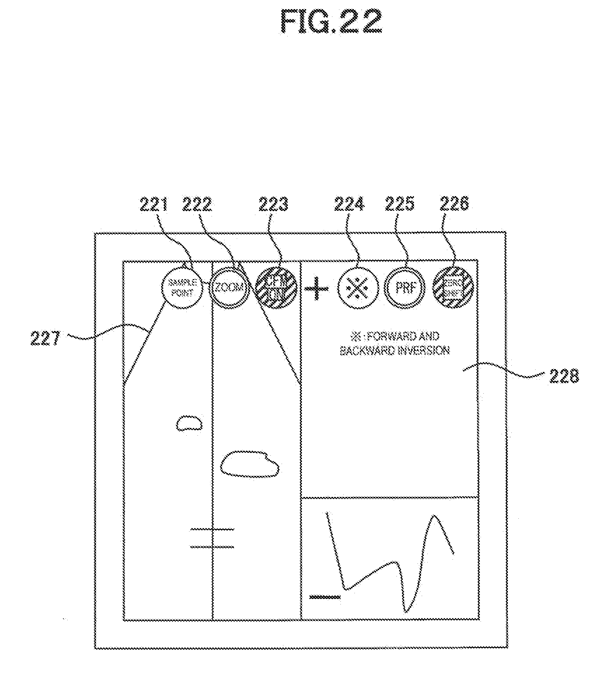

Fig. 22 shows guide display of touching operation on operation monitor 181.

On operation monitor 181, guide 221 ∼ guide 226 are displayed. Guide 221 ∼ guide 226 indicate the processing content for each kind of touch state.

As shown in Fig. 22, when the processing state is "Doppler mode real time", guide 221 ∼ guide 223 are displayed on the ultrasonic image 227 (B-mode image) side, and guide 224 ∼ guide 226 are displayed on Doppler image 228 side.

Guide 221 indicates the processing content upon single touch operation by a single circle, in concrete terms the movement of a Doppler sample point. Guide 222 indicates the processing content upon double-touch operation by a double circle, in concrete terms the zooming of a region of interest. Guide 223 indicates the processing content upon slide-touch operation by a colored circle such as in gray, in concrete terms that CFM/ROI (a region of interest of color flow mapping) is set in a slide-touched region.

Guide 224 indicates the processing content upon single-touch operation by a single circle, in concrete terms forward and backward inversion display of a Doppler image. Guide 225 indicates the processing content upon double-touch operation by a double circle, in concrete terms the change of repetition frequency (PRF). Guide 226 indicates the processing content upon slide-touch operation by a colored circle such as in gray, in concrete terms the shift of current velocity zero-level position.

While the function names are displayed on guide 221 ∼ guide 226 in Fig. 22, the marks that represent the functions may also be displayed.

In this manner, while the processing contents are different depending on the touch position, touch state or processing state in touching operation on touch panel 183 of operation monitor 181, it is possible to anticipate the respective processing contents in advance by using display guides. The operator does not have to memorize the processing contents for the respective touch position, touch state or processing state.

### (7-4. Display of a touch position)

Fig. 23 shows display of a touch position.

As shown in screen G2301, the operator touches ultrasonic image 231 using fingertip 232 on operation monitor 181 comprising touch panel 183. At the same time, as shown in screen G2302, on ultrasonic image 233 of observation monitor 182, touch position mark 234 is displayed at the position corresponding to the touch position of operation monitor 181.

In this manner, the touch position in operation monitor 181 can be confirmed even when the operator's eyes are fixed on observation monitor 182.

### (8. Concrete example of ultrasonic diagnostic apparatus 180)

Next, the case for using a portable device such as a notebook computer for ultrasonic diagnostic apparatus 180 will be described.

### (8-1. Setting an operation monitor in an operation unit)

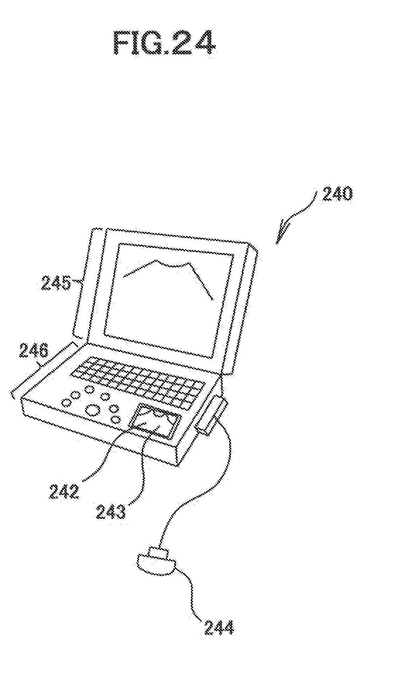

Fig. 24 is an external view of ultrasonic diagnostic apparatus 240.

Ultrasonic diagnostic apparatus 240 is configured having a notebook personal computer formed by display unit 245 such as a liquid crystal display and operation unit 246 such as a keyboard, being provided with a pointing device such as ultrasonic probe 244. Operation monitor 242 is provided in operation unit 246. On the surface of operation monitor 242, touch panel 243 is provided.

Operation monitor 242, touch panel 243 and display unit 245 in Fig. 24 are equivalent to operation monitor 181, touch panel 183 and observation monitor 182 in Fig. 18.

In this manner, ultrasonic diagnostic apparatus 240 is easy to carry and can be used regardless of the location. Also, the smudge of display unit 245 due to touching operation can be prevented by providing operation monitor 242 comprising touch panel 243.

### (8-2. Setting an operation monitor other than an operation unit and a display unit)

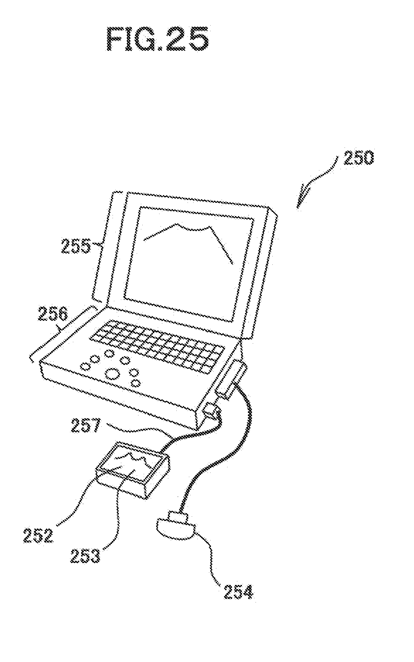

Fig. 25 is an external view of ultrasonic diagnostic apparatus 250.

Ultrasonic diagnostic apparatus 250 is configured having a notebook personal computer formed by display unit 255 such as a liquid crystal display and operation unit 256 such as a keyboard, being provided with a pointing device such as ultrasonic probe 254. Touch panel 253 is provided on the surface of operation monitor 252. Operation monitor 252 is provided individually from display unit 255 and operation unit 256. Operation unit 252 is, for example, connected to the main body by cable 257.

Operation monitor 252, touch panel 253 and display unit 255 in Fig. 25 are equivalent to operation monitor 181, touch panel 183 and observation monitor 182 in Fig. 18.

In this manner, by making operation monitor 252 capable of executing external connection, it is possible to use a multipurpose device such as a notebook personal computer without modification.

### (9. Effect of the second embodiment)

As described above, since touch-panel operation can be executed on the operation monitor without influencing the image observation on the observation monitor by providing the operation monitor comprising a touch panel separate from the observation monitor, it is possible to improve operability and diagnosis efficiency. Also, since the touch panel does not need to be attached to the observation monitor, there is no need to worry about the deterioration of image quality or display performance of the touch panel itself or the influence by smudge due to grease, etc. which adheres on the touch panel.

The observation monitor is disposed at the size, position and angle which are suitable for the image observation. The touch panel does not have to be provided on the observation monitor. The operation monitor comprising the touch panel is disposed at the size, position and angle which are suitable for the operation.

### (10. Other matters)

While preferred embodiments of the ultrasonic diagnostic apparatus related to the present invention have been described above referring to the attached diagrams, no limitations are intended to the examples therein. It is therefore evident to those skilled in the art that the particular embodiments disclosed above may be altered or modified without departing from the scope of the technical idea disclosed therein and all such variations are considered within the scope and spirit of the invention.

Also, the above-described first embodiment and second embodiment may be appropriately combined to configure an ultrasonic diagnostic apparatus.

## Claims

1. An ultrasonic diagnostic apparatus comprising:
an ultrasonic probe configured to transmit/receive an ultrasonic wave to/from an object to be examined;
an image processing unit configured to construct an ultrasonic image based on the ultrasonic reception signals outputted from the ultrasonic probe;
an image display unit configured to display the constructed ultrasonic image; and
a touch panel provided on the anterior surface of the image display unit,
**characterized in** further comprising:
a touch position detection unit configured to detect the touch position on the touch panel;
a touch state detection unit configured to detect the touch state on the touch panel;
a processing state detection unit configured to detect the current processing state; and
a processing determination unit configured to determine and execute the processing to be executed based on at least one of the detected touch position, touch state or processing state.

2. The ultrasonic diagnostic apparatus according to claim 1, **characterized in** further comprising an operation image creating unit configured to create an operation image for supporting the operation on the touch panel based on at least one of the detected touch position, touch state or processing state and to display the created image on the image display unit.

3. An ultrasonic diagnostic apparatus comprises:
an ultrasonic probe configured to transmit/receive an ultrasonic wave to/from an object to be examined;
an image processing unit configured to construct an ultrasonic image based on the ultrasonic reception signals outputted from the ultrasonic probe; and
a first image display unit configured to display the constructed ultrasonic image;
**characterized in** further comprising:
a second image display unit provided separate from the first image display unit, configured to display the ultrasonic image,
a touch panel to be provided on the anterior surface of the second image display unit;
a touch position detection unit configured to detect the touch position on the touch panel;
a touch state detection unit configured to detect the touch state on the touch panel;
a processing state detection unit configured to detect the current processing state; and
a processing determination unit configured to determine and execute the processing to be executed based on at least one of the detected touch position, touch state or processing state.

4. The ultrasonic diagnostic apparatus according to claim 3, **characterized in** further comprising an operation image creating unit configured to create an operation image for supporting the operation on the touch panel based on at least one of the detected touch position, touch state or processing state, and displays the created image on the first image display unit and/or the second image display unit.

5. The ultrasonic diagnostic apparatus according to claim 4, **characterized in that** the operation image displayed on the second image display unit is different from the operation image displayed on the first image display unit.

6. The ultrasonic diagnostic apparatus according to claim 3 ∼ 5, **characterized in that** the position corresponding to the touch position on the touch panel is made identifiable on the first image display unit.

7. The ultrasonic diagnostic apparatus according to claim 3 ∼ 6, wherein the second image display unit comprising the touch panel is disposed in the vicinity of the operation unit of an ultrasonic diagnostic apparatus.

8. The ultrasonic diagnostic apparatus according to claim 3 ∼ 6, **characterized in that** the second display unit comprising the touch panel is detachable or connectable by a connection wire to the main body of an ultrasonic apparatus.

9. The ultrasonic diagnostic apparatus according to claim 1 ∼ 8, **characterized in that** the processing to be determined by the processing determination unit includes at least one of the setting of a focusing position, setting of a region of interest, setting of a Doppler sample point, forward and backward inversion of Doppler image display, size setting of a region of interest, enlargement and reduction of an image, setting of attribution of the object and setting of parameter.

10. The ultrasonic diagnostic apparatus according to claim 1 ∼ 9, **characterized in that** a guide for indicating the correspondence between the touch position and the processing to be executed is displayed on the image display unit comprising the touch panel.

11. The ultrasonic diagnostic apparatus according to claim 1 ∼ 3, **characterized in that** the touch position is the one of the single touch in which the touch panel is touched one time, the double touch in which the touch panel is touched twice in succession or the slide touch in which a fingertip slides while touching the touch panel.

12. The ultrasonic diagnostic apparatus according to claim 11, wherein the processing determination unit determines different processing to be executed between the single touch, the double touch and the slide touch.
